(19) Europäisches Patentamt
European Patent Office
Office européen des brevets

(11) Veröffentlichungsnummer: **0 033 087 A2**

(12) **EUROPÄISCHE PATENTANMELDUNG**

(21) Anmeldenummer: **81100220.3**

(22) Anmeldetag: **14.01.81**

(51) Int. Cl.³: **C 07 D 249/08**
A 01 N 43/64

(30) Priorität: **24.01.80 DE 3002430**

(43) Veröffentlichungstag der Anmeldung:
**05.08.81 Patentblatt 81/31**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Kranz, Eckart, Dr.**
**Am Acker 9**
**D-5600 Wuppertal 1(DE)**

(72) Erfinder: **Büchel, Karl Heinz, Prof. Dr.**
**Dabringhausener Strasse 42**
**D-5093 Burscheid(DE)**

(72) Erfinder: **Frohberger, Paul-Ernst, Dr.**
**Willi-Baumeister-Strasse 5**
**D-5090 Leverkusen 1(DE)**

(54) **Triazolyl-alkandiol-Derivate, Verfahren zu ihrer Herstellung und ihre Verwendung als Fungizide.**

(57) Die Erfindung betrifft neue Triazolyl-alkandiol-Derivate, ein Verfahren zu ihrer Herstellung und ihre Verwendung als Fungizide.

Die neuen Verbindungen der allgemeinen Formel

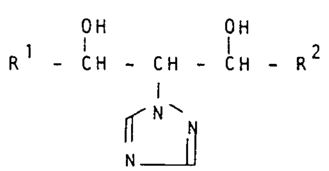

(I)

in welcher $R^1$ und $R^2$ die in der Beschreibung angegebene Bedeutung besitzen, werden erhalten, wenn man ω-Triazolyl-β-hydroxyketone nach bekannten Methoden in üblicher Weise reduziert und gegebenenfalls anschließend eine Säure oder ein Metallsalz addiert. Die Wirkstoffe sind für den Gebrauch als Pflanzenschutzmittel geeignet und können mit besonders gutem Erfolg zur Bekämpfung solcher Pilze eingesetzt werden, die echte Mehltauerkrankungen hervorrufen.

EP 0 033 087 A2

BAYER AKTIENGESELLSCHAFT
Zentralbereich
Patente, Marken und Lizenzen

5090 Leverkusen, Bayerwerk
Slr/W
Ia

# Triazolyl-alkandiol-Derivate, Verfahren zu ihrer Herstellung und ihre Verwendung als Fungizide

Die vorliegende Erfindung betrifft neue Triazolyl-alkandiol-Derivate, ein Verfahren zu ihrer Herstellung und ihre Verwendung als Fungizide.

Es ist bereits bekannt geworden, daß Triazolyl-O,N-acetale, wie beispielsweise im Phenylteil substituierte 3,3-Dimethyl-1-phenoxy-1-triazolyl-2-hydroxy-butane bzw. deren acylierte Derivate, im allgemeinen gute fungizide Eigenschaften aufweisen (vergleiche DE-OS 23 24 010 [LeA 14 971] und DE-OS 26 00 799 [LeA 16 838]). Die Wirkung ist jedoch, insbesondere bei niedrigen Aufwandmengen und -konzentrationen, in einigen Anwendungsbereichen nicht immer voll befriedigend.

Es wurden neue Triazolyl-alkandiol-Derivate der allgemeinen Formel

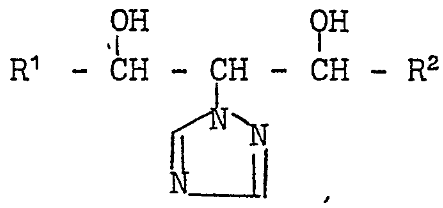

(I)

,

in welcher

$R^1$ für gegebenenfalls substituiertes Alkyl oder gegebenenfalls substituiertes Phenyl steht und

$R^2$ für Halogenalkyl, Halogenalkenyl oder Alkoxycarbonyl steht,

und deren physiologisch verträglichen Säureadditions-Salze und Metallsalz-Komplexe gefunden.

Die erfindunggemäßen Verbindungen der Formel (I) enthalten asymmetrische Kohlenstoffatome; sie können in verschiedenen Diastereomeren vorliegen. Sie fallen im allgemeinen als Diastereomerengemische unterschiedlicher Zusammensetzung an. In allen Fällen liegen sie vorwiegend als Racemate vor.

Weiterhin wurde gefunden, daß man die Triazolyl-alkandiol-Derivate der Formel (I) erhält, wenn man ω-Triazolyl-ß-hydroxy-ketone der Formel

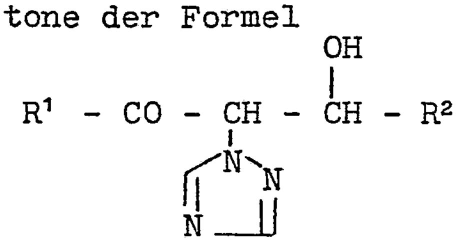

(II)

in welcher

$R^1$ und $R^2$ die oben angegebene Bedeutung haben,

nach bekannten Methoden in üblicher Weise reduziert und gegebenenfalls anschließend eine Säure oder ein Metallsalz addiert.

Die neuen Triazolyl-alkandiol-Derivate weisen starke fungizide Eigenschaften auf. Dabei zeigen überraschenderweise die erfindungsgemäßen Verbindungen eine höhere Wirkung als die aus dem Stand der Technik bekannten substituierten 3,3-Dimethyl-1-phenoxy-1-triazolyl-2-hydroxy-butane bzw. deren acylierten Derivate, welches naheliegende Verbindungen gleicher Wirkungsrichtung sind. Die erfindungsgemäßen Stoffe stellen somit eine Bereicherung der Technik dar.

Von den erfindungsgemäßen Triazolyl-alkandiol-Derivaten sind bevorzugt diejenigen, in denen $\underline{R^1}$ für gegebenenfalls substituiertes geradkettiges oder verzweigtes Alkyl mit 1 bis 4 Kohlenstoffatomen steht, wobei als Substituenten vorzugsweise infrage kommen: Halogen, wie insbesondere Fluor, Chlor und Brom; Alkylcarbonyloxy mit 1 bis 4 Kohlenstoffatomen im Alkylteil; gegebenenfalls substituiertes Phenylcarbonyloxy, wobei als Substituenten vorzugsweise genannt seien: Halogen, wie insbesondere Fluor, Chlor und Brom, Alkyl mit 1 bis 2 Kohlenstoffatomen, Halogenalkyl mit 1 bis 2 Kohlenstoff- und 1 bis 5 Halogenatomen, insbesondere Fluor und Chlor, sowie Cyano oder Nitro; Alkyl- oder Dialkyl-carbamoyloxy mit 1 bis 4 Kohlenstoffatomen in jedem Alkylteil; Alkyl-sulfonyloxy mit 1 bis 4 Kohlenstoffatomen oder gegebenenfalls substituiertes Phenylsulfonyloxy, wobei als Phenylsubstituenten vorzugsweise die bereits oben genannten infrage kommen; Dialkylaminosulfonyloxy mit 1 bis 4 Kohlenstoffatomen in jedem Alkylteil; Alkoxy mit 1 bis 4 Kohlenstoffatomen sowie gegebenenfalls substituiertes Phenoxy, wobei als Substituenten vorzugsweise die bereits oben genannten infrage kommen. $R^1$ steht außerdem bevorzugt für gegebenenfalls einfach oder mehrfach, gleich oder verschieden substituiertes Phenyl, wobei als Substituenten vorzugsweise infrage kommen: Halogen, wie insbesondere Fluor, Chlor oder Brom; Cyano, Nitro, geradkettiges oder verzweigtes Alkyl mit bis zu 4 Kohlenstoffatomen, Cycloalkyl mit 5 bis 7 Kohlenstoffatomen, wie insbesondere Cyclohexyl, Halogenalkyl mit bis zu 2 Kohlenstoff- und bis zu 5 Halogenatomen, wie insbesondere Fluor- und Chloratomen, sowie gegebenenfalls durch Halogen, insbesondere Fluor und Chlor, Cyano oder Nitro substituiertes Phenyl, Phenoxy oder Benzyl.

$\underline{R}^2$ steht vorzugsweise für geradkettiges oder verzweigtes Halogenalkyl mit 1 bis 4 Kohlenstoffatomen und 1 bis 5 Halogenatomen, wie insbesondere Fluor, Chlor oder Brom; geradkettiges oder verzweigtes Halogenalkenyl mit 2 bis 4 Kohlenstoffatomen und 1 bis 5 Halogenatomen, wie insbesondere Fluor, Chlor oder Brom; sowie für Alkoxycarbonyl mit 1 bis 4 Kohlenstoffatomen im Alkoxyteil.

Ganz besonders bevorzugt sind diejenigen Verbindungen der Formel (I), in denen $\underline{R}^1$ für tert.-Butyl, sek.-Butyl, Isopropyl, Chlor-tert.-butyl, Brom-tert.-butyl. Fluor-tert.-butyl, 1,3-Dichlor-2-methyl-prop-2-yl, 2-Methylcarbamoyloxy-prop-2-yl, 2-Diethylcarbamoyloxy-prop-2-yl, Methylsulfonyloxy-tert.-butyl, Phenylsulfonyloxy-tert.-butyl, 4-Chlorphenylsulfonyloxy-tert.-butyl, Acetoxy-tert.-butyl, Ethylcarbonyloxy-tert.-butyl, Phenylcarbonyloxy-tert.-butyl, 4-Chlorphenylcarbonyloxy-tert.-butyl, Diethylaminosulfonyloxy-tert.-butyl, Methoxy-tert.-butyl, Ethoxy-tert.-butyl, Isopropoxy-tert.-butyl, Phenoxy-tert.-butyl und 4-Chlorphenoxy-tert.-butyl steht; ferner für Phenyl, Chlorphenyl, Dichlorphenyl, Chlor-methyl-phenyl, Bromphenyl, Nitrophenyl, Biphenylyl, Nitrobiphenylyl, Chlorbiphenylyl, Phenoxyphenyl, Chlorphenoxyphenyl, Benzylphenyl. oder Chlorbenzylphenyl steht; $\underline{R}^2$ für Trichlormethyl, Dichlorfluormethyl, Trifluormethyl, Dichlormethyl, Chlormethyl, 1,1,2-Trichlorethyl, 1,1-Dibromethyl, 1,1-Dichlorethyl, 1,1,2-Trichlor-propyl, 2-Chlor-prop-2-yl, 1,2,2,-Trichlorvinyl; 2,2-Dichlorvinyl, Methoxycarbonyl oder Ethoxycarbonyl steht.

Im einzelnen seien außer den bei den Herstellungsbeispielen angegebenen Verbindungen die folgenden Verbindungen der allgemeinen Formel (I) genannt:

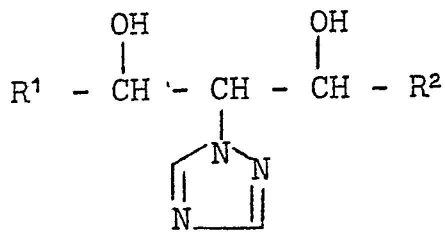

(I)

| $R^1$ | $R^2$ |
|---|---|
| $-C(CH_3)_3$ | $-CHCl_2$ |
| $-C(CH_3)_3$ | $-CO-OCH_3$ |
| $-C(CH_3)_3$ | $-CO-OC_2H_5$ |
| $-C(CH_3)_3$ | $-CO-OC_4H_9$ |
| $-C(CH_3)_3$ | $-CCl=CCl_2$ |
| $-C(CH_3)_3$ | $-CCl_2-CH_2Cl$ |
| $-C(CH_3)_2-CH_2Cl$ | $-CCl_2-CHCl-CH_3$ |
| $-C(CH_3)_2-CH_2Cl$ | $-CO-OCH_3$ |
| $-C(CH_3)_2-CH_2Cl$ | $-CCl_2-CH_2Cl$ |
| $-C(CH_3)_2-CH_2Cl$ | $-CO-OC_2H_5$ |
| $-C(CH_3)_2-CH_2Cl$ | $-CCl(CH_3)_2$ |
| $-C(CH_3)_2-CH_2Cl$ | $-CH=CCl_2$ |
| $-C(CH_3)_2-CH_2Cl$ | $-CCl=CCl_2$ |
| $-C(CH_3)_2-CH_2Br$ | $-CCl_3$ |
| $-C(CH_3)_2-CH_2Br$ | $-CO-OCH_3$ |
| $-C(CH_3)_2-CH_2Br$ | $-CH=CCl_2$ |
| $-C(CH_3)_2-CH_2Br$ | $-CCl=CCl_2$ |
| $-C(CH_3)_2-CH_2Br$ | $-CCl(CH_3)_2$ |
| $-C(CH_3)_2-CH_2Br$ | $-CHCl_2$ |
| $-C(CH_3)_2-CH_2Br$ | $-CCl_2-CHCl-CH_3$ |
| $-C(CH_3)_2-CH_2Br$ | $-CO-OC_2H_5$ |

| $R^1$ | $R^2$ |
|---|---|
| $-C(CH_3)_2-CH_2F$ | $-CO-OCH_3$ |
| $-C(CH_3)_2-CH_2F$ | $-CCl(CH_3)_2$ |
| $-C(CH_3)_2-CH_2F$ | $-CCl_2-CH_2Cl$ |
| $-C(CH_3)_2-CH_2F$ | $-CO-OC_2H_5$ |
| $-C(CH_3)_2-CH_2F$ | $-CH=CCl_2$ |
| $-C(CH_3)_2-CH_2F$ | $-CCl=CCl_2$ |
| 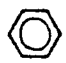 | $-CCl_3$ |
| 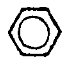 | $-CCl_2CHCl-CH_3$ |
| 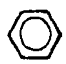 | $-CO-OC_2H_5$ |
| 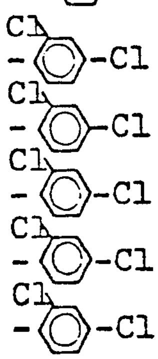 | $-CCl_2-CHCl-CH_3$ |
| | $-CO-OC_2H_5$ |
| | $-CCl_2-CHCl-CH_3$ |
| | $-CH=CCl_2$ |
| | $-CCl=CCl_2$ |
| 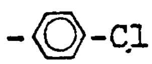 | $-CCl_2-CH_2Cl$ |
| 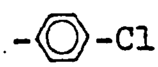 | $-CO-OC_2H_5$ |
| 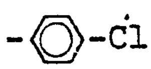 | $-CCl_2-CHCl-CH_3$ |
| 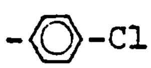 | $-CH=CCl_2$ |
| 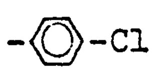 | $-CCl=CCl_2$ |
| 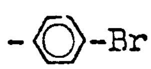 | $-CCl_3$ |
| 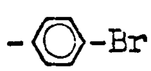 | $-CO-OC_2H_5$ |
| 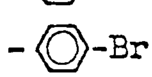 | $-CCl_2-CHCl-CH_3$ |
| 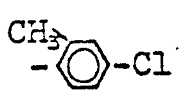 | $-CCl_3$ |

| $R^1$ | $R^2$ |
|---|---|
| 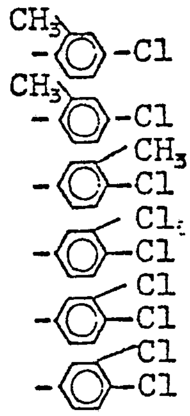 | $-CO-OC_2H_5$ |
| | $-CCl_2-CHCl-CH_3$ |
| | $-CCl_3$ |
| | $-CCl_3$ |
| | $-CO-OC_2H_5$ |
| | $-CCl_2-CHCl-CH_3$ |
| 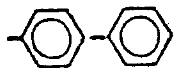 | $-CCl_3$ |
| 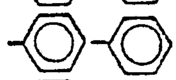 | $-CCl_2-CHCl-CH_3$ |
| 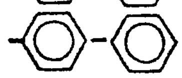 | $-CO-OC_2H_5$ |
| 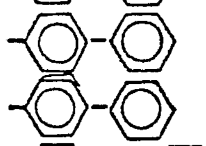 | $-CH=CCl_2$ |
| | $-CCl=CCl_2$ |
| 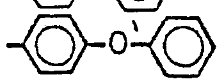 | $-CCl_3$ |
| 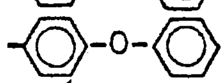 | $-CO-OC_2H_5$ |
| 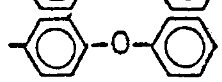 | $-CCl_2-CHCl-CH_3$ |
| 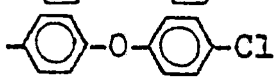 | $-CO-OC_2H_5$ |
| 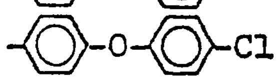 | $-CCl_2-CHCl-CH_3$ |
| 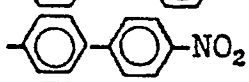 | $-CCl_3$ |
| 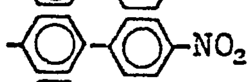 | $-CO-OC_2H_5$ |
| 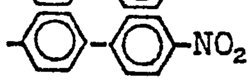 | $-CCl_2-CHCl-CH_3$ |
| $-C(CH_3)_2-O-CO-NHCH_3$ | $-CCl_3$ |
| $-C(CH_3)_2-O-CO-NHCH_3$ | $-CO-OC_2H_5$ |
| $-C(CH_3)_3-O-CO-NHCH_3$ | $-CCl_2-CHCl-CH_3$ |

| $R^1$ | $R^2$ |
|---|---|
| $-C(CH_3)_2-CH_2-O-SO_2CH_3$ | $-CCl_3$ |
| $-C(CH_3)_2-CH_2-O-SO_2CH_3$ | $-CO-OC_2H_5$ |
| $-C(CH_3)_2-CH_2-O-SO_2CH_3$ | $-CCl_2-CHCl-CH_3$ |
| $-C(CH_3)_2-CH_2-O-SO_2CH_3$ | $-CCl_2-CH_2Cl$ |
| $-C(CH_3)_2-CH_2-O-SO_2-$ 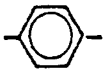 $-CH_3$ | $-CCl_3$ |
| $-C(CH_3)_2-CH_2-O-SO_2-$ 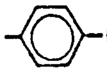 $-CH_3$ | $-CO-OC_2H_5$ |
| $-C(CH_3)_2-CH_2-O-SO_2-$ 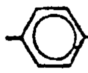 $-CH_3$ | $-CCl_2-CHCl-CH_3$ |
| $-C(CH_3)_2-CH_2-O-CO-CH_3$ | $-CCl_3$ |
| $-C(CH_3)_2-CH_2-O-CO-CH_3$ | $-CO-OC_2H_5$ |
| $-C(CH_3)_2-CH_2-O-CO-CH_3$ | $-CCl_2-CHCl-CH_3$ |
| $-C(CH_3)_2-OCH_3$ | $-CCl_3$ |
| $-C(CH_3)_2-OCH_3$ | $-CO-OC_2H_5$ |
| $-C(CH_3)_2-OCH_3$ | $-CCl_2-CHCl-CH_3$ |
| $-C(CH_3)_2-OC_2H_5$ | $-CCl_3$ |
| $-C(CH_3)_2-OC_2H_5$ | $-CO-OC_2H_5$ |
| $-C(CH_3)_2-OC_2H_5$ | $-CCl_2-CHCl-CH_3$ |
| $-C(CH_3)_2-O-$ 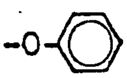 | $-CCl_3$ |
| $-C(CH_3)_2-O-$ 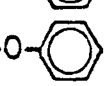 | $-CO-O-C_2H_5$ |
| $-C(CH_3)_2-O-$ 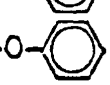 | $-CCl_2-CHCl-CH_3$ |

Verwendet man beispielsweise 1,1,1-Trichlor-2-hydroxy-3-(1,2,4-triazol-1-yl)-5,5-dimethyl-hexan-4-on und Natriumborhydrid als Ausgangsstoffe, so kann der Reaktionsablauf durch das folgende Formelschema wiedergegeben werden:

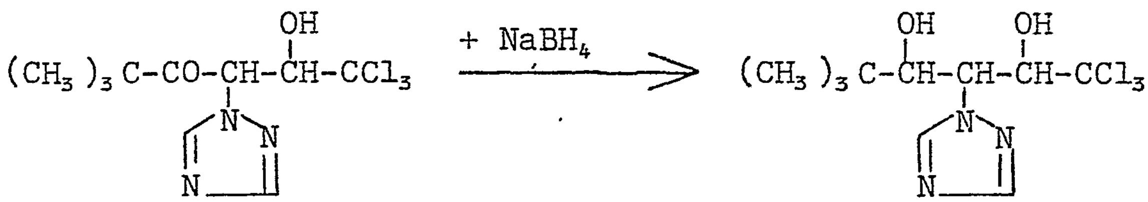


Die für das erfindungsgemäße Verfahren als Ausgangsstoffe zu verwendenden α-Triazolyl-ß-hydroxy-ketone sind durch die Formel (II) allgemein definiert. In dieser Formel stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden.

Die α-Triazolyl-ß-hydroxy-ketone der Formel (II) sind noch nicht bekannt. Sie sind jedoch zum Teil Gegenstand von eigenen älteren Anmeldungen, die noch nicht veröffentlicht sind (vergleiche Deutsche Patentanmeldung P 28 32 233.0 /‾Le A 18 970_7 vom 21.7.1978 und P 29 44 447.1 /‾Le A 19 990_7 vom 3.11.1979). Sie werden erhalten, indem man α-Triazolyl-ketone der Formel

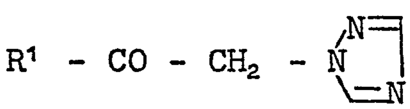
(III)

in welcher

$R^1$ die oben angegebene Bedeutung hat,

mit Aldehyden der Formel

$R^2$ - CHO (IV)

in welcher

$R^2$ die oben angegebene Bedeutung hat,

in Gegenwart eines inerten organischen Lösungsmittels, wie beispielsweise Methylenchlorid und insbesondere Eisessig, und in Gegenwart eines Katalysators, wie beispielsweise Titantetrachlorid und insbesondere Natriumacetat, bei Temperaturen zwischen 20 und 100°C umsetzt (vergleiche auch die Herstellungsbeispiele).

Die α-Triazolyl-ketone der Formel (III) sind teilweise bekannt (vergleiche DE-OS 20 63 857 und DE-OS 24 31 407 [LeA 15 735]). Die noch nicht bekannten können nach den dort beschriebenen Verfahren erhalten werden, indem man z.B. entsprechende α-Halogenketone mit 1,2,4-Triazol in Gegenwart eines inerten organischen Lösungsmittels, wie Aceton, und in Gegenwart eines Säurebindemittels, wie beispielsweise Natriumcarbonat, vorzugsweise in der Siedehitze umsetzt (vergleiche auch die Herstellungsbeispiele).

Die erfindungsgemäße Reduktion erfolgt in üblicher Weise, wie z.B. durch Umsetzung mit komplexen Hydriden, gegebenenfalls in Gegenwart eines Verdünnungsmittels; oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Gegebenenfalls kann auch Wasser als Lösungsmittel verwendet werden, vorzugsweise in Mischung mit einem polaren organischen Lösungsmittel. Die Reaktion wird im allgemeinen bei 0 bis 30°C, vorzugsweise bei 0 bis 20°C durchgeführt. Hierzu setzt man auf 1 Mol des Ketones der Formel (II) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (II) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren,wie z.B. p-Toluolsulfonsäure und 1.5-Naphthalindisulfonsäure.
Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.
Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.
Die Metallsalz-Komplexe von Verbindungen der Formel (I) können in einfacher Weise nach üblichen Verfahren erhalten werden, so z.B.durch Lösen des Metallsalzes in Alkohol,z.B.Ethanol und Hinzufügen zur Verbindung der Formel (I).Man kann Metallsalz-Komplexe in bekannter Weise,z.B. durch Abfiltrieren,Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gurkenmehltaus (Erysiphe cichoreacearum) oder des Gerstenmehltaus bzw. des Getreidemehltaus (Erysiphe graminis); sowie zur Bekämpfung solcher Pilze, die Schorfkrankheiten hervorrufen; so zur Bekämpfung von Venturia-Arten, wie z.B. gegen den Erreger des Apfelschorfs (Fusicladium dendriticum). Besonders hervorzuheben ist, daß die erfindungsgemäßen Wirkstoffe nicht nur eine protektive Wirkung entfalten, sondern auch systemisch wirksam

sind. So gelingt es, Pflanzen gegen Pilzbefall zu schützen, wenn man den Wirkstoff über den Boden und die Wurzel oder über das Saatgut den oberirdischen Teilen der Pflanze zuführt.

In bestimmten Aufwandmengen zeigen die erfindungsgemäßen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

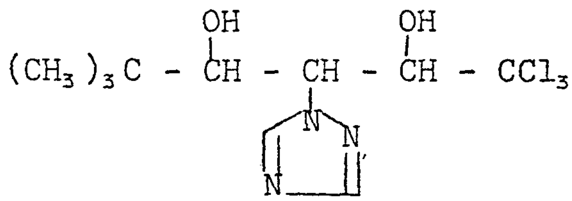

Zu einer Lösung von 31,5g (0,1 Mol) 1,1,1-Trichlor-2-hydroxy-3-(1,2,4-triazol-1-yl)-5,5-dimethyl-hexan-4-on in 70 ml Wasser und 75 ml Dioxan werden bei Raumtemperatur unter Eiskühlung 4,4g (0,12 Mol) Natriumborhydrid in 30ml Wasser langsam zugetropft. Man läßt 10 Stunden bei Raumtemperatur nachrühren, versetzt mit 25 ml konzentrierter Salzsäure und läßt erneut ca. 2 Stunden bei Raumtemperatur nachrühren. Anschließend wird das Reaktionsgemisch in 500 ml gesättigte Natriumhydrogencarbonatlösung eingerührt. Man extrahiert dreimal mit je 100 ml Methylenchlorid, trocknet die vereinigten organischen Phasen über Natriumsulfat und engt durch Abdestillieren des Lösungsmittels im Vakuum ein. Der Rückstand wird in Methylenchlorid aufgenommen und mit Aktivkohle unter Rückfluß erhitzt. Das Gemisch wird filtriert und das Filtrat eingeengt. Man erhält 19,0g (60 % der Theorie) 1,1,1-Trichlor-(1,2,4-triazol-1-yl)-5,5-dimethyl-hexan-2,4-diol vom Schmelzpunkt 83-113°C.

Herstellung der Vorstufen

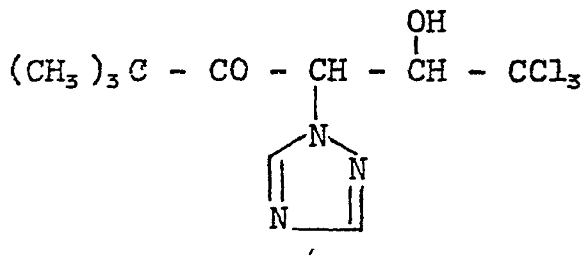

33,4g (0,2 Mol) 3,3-Dimethyl-1-(1,2,4-triazol-1-yl)-butan-2-on werden in 150 ml Methylenchlorid gelöst und auf -5°C abgekühlt. Dazu tropft man langsam 19g (0,1 Mol) Titantetrachlorid und anschließend 29,5g (0,2 Mol) Chloral. Während der Zudosierung soll die Innentemperatur bei -5°C konstant bleiben. Danach wird langsam bis zum Rückfluß erwärmt und 3 Stunden verrührt. Die Reaktionslösung wird auf Eis gegossen und der entstehende weiße käsige Niederschlag abgesaugt. Nach Auskochen in 250 ml Methanol und anschließender Trocknung erhält man 56g (90 % der Theorie) 1,1,1-Trichlor-2-hydroxy-3-(1,2,4-triazol-1-yl)-5,5-dimethyl-hexan-4-on vom Schmelzpunkt 220-222°C.

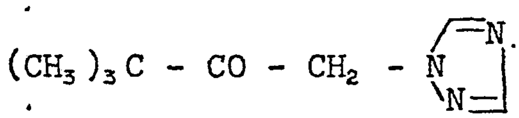

138g (2 Mol) 1,2,4-Triazol werden bei Raumtemperatur portionsweise zu 276,4g (2 Mol) gemahlenem Kaliumcarbonat und 269,2g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8g (72 % der Theorie) 3,3-Dimethyl-1-(1,2,4-triazol-1-yl)-butan-2-on vom Schmelzpunkt 62-64°C.

Beispiel 2

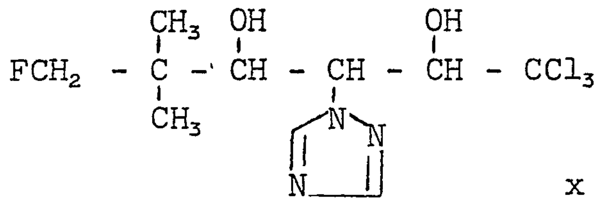

x $CuCl_2$

6,7g (0,02 Mol) 1,1,1-Trichlor-3-(1,2,4-triazol-1-yl)-5,5-dimethyl-6-fluor-hexan-2,4-diol (Beispiel 7; Herstellung gemäß Beispiel 1) werden in 50 ml Ethanol suspendiert und mit 3,4g (0,02 Mol) Kupfer-II-chlorid-dihydrat in 50 ml Ethanol 30 Minuten lang bei Raumtemperatur verrührt. Dabei erhält man eine klare, grüne Lösung, die durch Abdestillieren des Lösungsmittels im Vakuum eingeengt wird. Man erhält 8,7g (93 % der Theorie) 1,1,1-Trichlor-3-(1,2,4-triazol-1-yl)-5,5-dimethyl-6-fluor-hexan-2,4-diol-kupfer-II-chlorid vom Schmelzpunkt 131-40°C.

In entsprechender Weise und gemäß dem erfindungsgemäßen Verfahren werden die nachfolgenden Beispiele der allgemeinen Formel

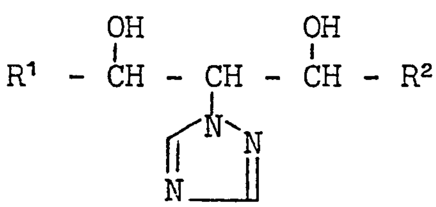

(I)

erhalten:

| Bsp. Nr. | $R^1$ | $R^2$ | Schmelz-punkt(°C) |
|---|---|---|---|
| 3 | $-C(CH_3)_3$ | $-CF_3$ | 134-44 |
| 4 | $-C(CH_3)_3$ | $-CCl(CH_3)_2$ | 120-22 |
| 5 | $-C(CH_3)_3$ | $-CCl_2-CH_2Cl$ | 57-63 |
| 6 | $-C(CH_3)_3$ | $-CH=CCl_2$ | Oel |

| Bsp. Nr. | $R^1$ | $R^2$ | Schmelzpunkt (°C) |
|---|---|---|---|
| 7 | $-C(CH_3)_2-CH_2F$ | $-CCl_3$ | Oel |
| 8 | $-C(CH_3)_2-CH_2Cl$ | $-CCl_3$ | 57-68 |
| 9 | $-C(CH_3)_2-CH_2F$ | $-CHCl_2$ | Oel |
| 10 | $-C(CH_3)_2-CH_2Cl$ | $-CHCl_2$ | 53-64 |
| 11 | $-C(CH_3)_2-CH_2F$ | $-CCl_2-CHCl-CH_3$ | Oel |
| 12 | 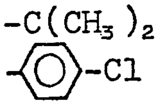 | $-CCl_3$ | 92-125 |
| 13 | 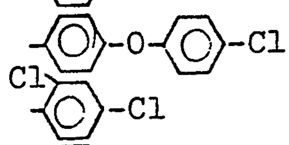 | $-CCl_3$ | Oel |
| 14 |  | $-CCl_3$ | 54-68 |
| 15 | 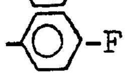 | $-CCl_3$ | Oel |
| 16 | $-C(CH_3)_3$ | $-COOCH_3$ | 82-84 |
| 17 | $-C(CH_3)_3$ | $-CCl_3$ | 162-69 ($xCuCl_2$) |
| 18 | $-C(CH_3)_3$ | $-CCl_3$ | 204-05 ($x2CuCl_2$) |
| 19 | $-C(CH_3)_3$ | $-CCl_2-CH_2Cl$ | 110-14 ($xCuCl_2$) |
| 20 | 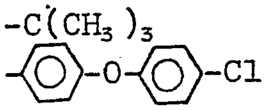 | $-CCl_3$ | 74-102 ($xCuCl_2$) |
| 21 | $-C(CH_3)_3$ | $-CCl_2-CHCl-CH_3$ | 54-57 |

Verwendungsbeispiele

In den nachfolgenden Beispielen werden die nachstehend angegebenen Verbindungen als Vergleichssubstanzen eingesetzt:

(A) 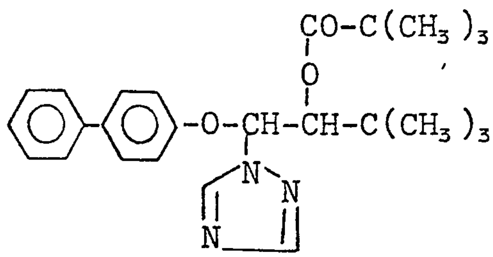

(B) 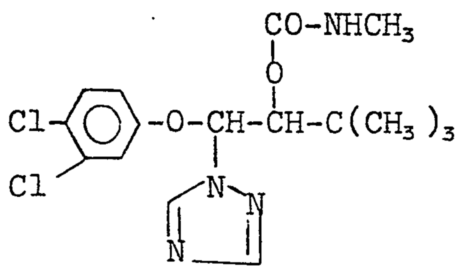

(C) 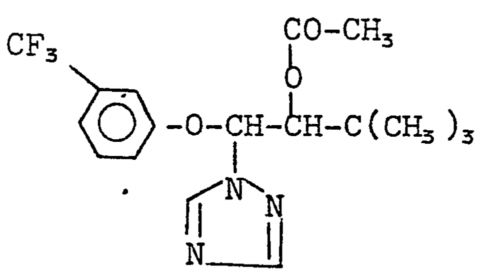

(D) 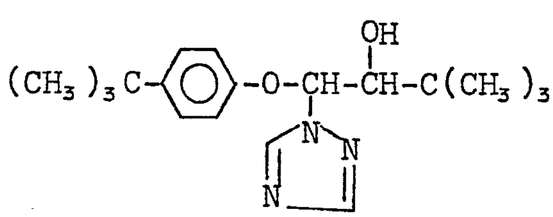

Beispiel A

Sproßbehandlungs-Test/ Getreidemehltau/ Protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.
Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.
Nach 6 Tagen Verweilzeit der Pflanzen bei einer Temperatur von 21-22°C und einer Luftfeuchtigkeit von 80-90% wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.
Bei diesem Test zeigen z.B. die folgenden Verbindungen eine sehr gute Wirkung, die derjenigen der aus dem Stand der Technik bekannten Verbindungen(A),(B) und (C) überlegen ist:
Verbindungen gemäß Herstellungsbeispielen 1,21,12,8,7, 13, 5 und 14.

Beispiel B

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel.Luftfeuchte und 16-stündiger Belichtung weiter kultiviert. Innerhalb von 6 Tagen bilden sich an den Blättern die typischen Mehltaupusteln aus.
Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z.B. die folgenden Verbindungen eine sehr gute Wirkung, die derjenigen der aus dem Stand der Technik bekannten Verbindung (D) überlegen ist:
Verbindungen gemäß Herstellungsbeispielen: 1,21 und 12.

Patentansprüche

1) Triazolyl-alkandiol-Derivate der allgemeinen Formel

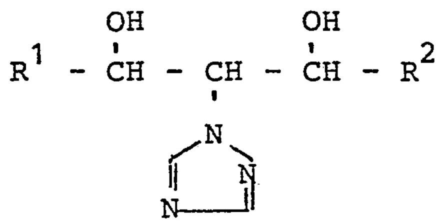

(I)

in welcher

$R^1$ für gegebenenfalls substituiertes Alkyl oder gegebenenfalls substituiertes Phenyl steht und

$R^2$ für Halogenalkyl, Halogenalkenyl oder Alkoxycarbonyl steht,

und deren physiologisch verträglichen Säureadditions-Salze und Metallsalz-Komplexe.

2) Verfahren zur Herstellung von Triazolyl-alkandiol-Derivaten, dadurch gekennzeichnet, daß man ω-Triazolyl-ß-hydroxy-ketone der Formel

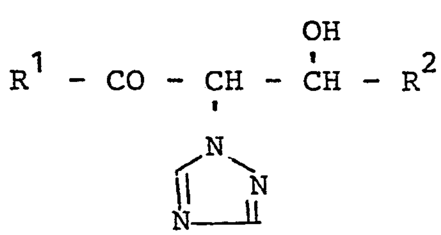

(II)

in welcher

$R^1$ und $R^2$ die in Anspruch 1 angegebene Bedeutung haben,

nach bekannten Methoden in üblicher Weise reduziert und gegebenenfalls anschließend eine Säure oder ein Metallsalz addiert.

3) Fungizide Mittel, gekennzeichnet durch einen Gehalt an mindestens einem Triazolyl-alkandiol-Derivat der Formel I.

4) Verfahren zur Bekämpfung von Pilzen, dadurch gekennzeichnet, daß man Triazolyl-alkandiol-Derivate der Formel I auf Pilze oder ihren Lebensraum einwirken läßt.

5) Verwendung von Triazolyl-alkandiol-Derivaten der Formel I zur Bekämpfung von Pilzen.

6) Verfahren zur Herstellung von fungiziden Mitteln, dadurch gekennzeichnet, daß man Triazolyl-alkandiol-Derivate der Formel I mit Streckmitteln und/oder oberflächenaktiven Mitteln vermischt.